# EUROPEAN PATENT APPLICATION

(11) **EP 2 357 003 A2**
(43) Date of publication of application: **17.08.2011**
(21) Application number: 09829350.9
(22) Date of filing: 27.11.2009
(51) Int. Cl.: A61K 39/395, A61P 35/00, A61K 33/24

(54) **ANTICANCER COMPOSITION COMPRISING ANTITUMOR AGENT AND SUBSTANCE HAVING INHIBITORY EFFECTS ON L1CAM ACTIVITY AND EXPRESSION**

(30) Priority: 27.11.2008 KR 20080118921
(71) Applicant: Korea Research Institute of Bioscience and Biotechnology, Yuseong-gu, Daejeon 305-333 (KR)
(72) Inventor: HONG, Hyo Jeong, Daejeon 305-370 (KR); MIN, Jeong-Ki, Chuncheon-si Gangwon-do 200-765 (KR); YOON, Hyunho, Cheongju-si Chungcheongbuk-do 361-792 (KR); LEE, Jung Whoi, Seoul 121-210 (KR)
(74) Representative: Gassner, Birgitta
(86) International application number: PCT/KR2009/007056
(87) International publication number: WO 2010/062143

(57) **Abstract**

The present invention relates to an anticancer composition comprising an antitumor agent as well as a substance having inhibitory effects on the activity or expression of L1CAM; more particularly, to an anticancer composition, which comprises an anti-L1CAM antibody specific to L1CAM, serving as a substance for inhibiting the activity of L1CAM; an oligonucleotide inhibiting the generation of L1CAM, serving as a substance for inhibiting the expression of L1CAM; and a substance selected from cisplatin, gemcitabine, 5-fluorouracil and taxol, serving as an antitumor agent. The composition according to the present invention has the benefit of the combined use of a substance having inhibitory effects on the activity or an expression of L1CAM and an antitumor agent concurrently, separately or sequentially so that it demonstrates stronger and more significant pharmaceutical effects than the exclusive use of those substances on inhibiting proliferation and inducing apoptosis in cancer cells, thereby making it very useful for cancer treatment.

<110>    Korea Research Institute of Bioscience and Biotechnology

<120>    A composition for treating L1CAM-expressing cancer comprising an
         inhibitor of activity or expression of L1CAM and anticancer agent

<130>    OPA09111PCT

<150>    KR10-2008-0118921
<151>    2008-11-27

<160>    2

<170>    KopatentIn 1.71

<210>    1
<211>    21
<212>    DNA
<213>    Artificial Sequence

<220>
<223>    L1CAM specific siRNA


<400>    1
gccaatgcct acatctacgt t                                                  21


<210>    2
<211>    19
<212>    DNA
<213>    Artificial Sequence

<220>
<223>    L1CAM non specific siRNA


<400>    2
cagtcgcgtt tgcgactgg                                                     19

## Description

### Technical Field

The present invention relates to an anticancer composition comprising an inhibitor of LICAM activity or expression and an antitumor agent. More specifically, the present invention relates to an anticancer composition comprising an anti-L1CAM antibody specific for L1CAM as an inhibitor of LICAM activity or oligonucleotide inhibiting expression of L1CAM as an inhibitor of L1CAM expression, and an antitumor agent selected from cisplatin, gemcitabine, 5-fluorouracil and taxol, wherein the composition exhibits greater synergistic therapeutic effects than exclusive administration of each of two substances and thus efficiently treats cancer, in particular, cancer expressing L1CAM.

### Background Art

L1 cell adhesion molecule (L1CAM) is an integral membrane glycoprotein which belongs to the immunoglobulin superfamily of cell adhesion molecules (CAMs) that mediate cell-to-cell adhesion on the cell surface and a molecular weight is 220 kDa. L1CAM was originally found in neurons and is known to perform neural migration, neurite outgrowth and cell migration. L1CAM is known to be expressed in normal tissues as well as nerve tissues and is being recently detected in several types of cancer cells.

Association between L1CAM and a variety of cancers has been reported. For example, L1CAM was reported to be expressed in a variety of tumors such as melanoma, neuroblastoma, ovarian carcinoma and colorectal carcinoma (Takeda, et al., J. Neurochem. 66:2338-2349, 1996; Thies et al, Eur. J. Cancer, 38:1708-1716, 2002; Arlt et al., Cancer Res. 66:936-943, 2006; Gavert et al., J. Cell Biol. 168:633-642, 2005). L1CAM has been found to be extracellularly secreted as cleavage products as well as membrane-bound forms (Gutwein et al., FASEP J. 17(2):292-4, 2003). In recent years, L1CAM was searched as one molecule which plays an important role in the growth of cancer cells (Primiano, et al., Cancer Cell. 4(1):41-53 2003) and emerged as a novel target in cancer treatment (US2004/0115206 A1, filed on 2004.6.17). Recently, antibodies to L1CAM have been found to inhibit growth and metastasis of ovarian cancer cells (Arlt, et al., Cancer Res. 66:936-943. 2006).

EP Patent Application No. 1172654 and US Patent Application Publication No. 2004/0259084 disclose a method for the diagnosis and prognosis of ovarian or endometrial tumors, characterized in that the L1CAM level is determined in a patient sample on the basis that presence of L1CAM is an indication of the presence of an ovarian or endometrial tumor or a possible predisposition for such a tumor, and a method of treating ovarian or endometrial tumors in a patient in need of such treatment, comprising administering to the patient a sufficient amount of a L1CAM antibody or a fragment thereof conjugated to a cytotoxic drug. However, the referenced patents above only disclose that L1CAM protein is a marker specific for ovarian or endometrial tumors in serum or tissues.

US Patent Publication No. 2004/0115206 discloses a reagent for inducing cell death in tumor cells such as breast cancer, colorectal cancer and cervical cancer using an antibody specifically binding to L1CAM, use of the antibody for cell death, and a pharmaceutical composition comprising the L1CAM antibody. As noted in this patent, inhibition of cell growth and induction of cell death can be carried out by bringing the tumor cells into contact with an effective amount of anti-L1CAM antibody sufficient for inhibiting cell growth or inducing cell death in the tumor cells.

International Patent Application No. PCT/EP2005/008148 discloses an L1CAM protein overexpressed in ovarian and endometrial carcinoma, a pharmaceutical composition for inhibiting the expression of L1CAM, and a method for preventing and treating ovarian and endometrial carcinoma using the composition. This patent discloses that the pharmaceutical composition comprising an anti-L1CAM antibody or a derivative thereof suppresses functions of ovarian and endometrial carcinoma and blocks the migration and growth of the cancer cells, thus realizing treatment of cancer.

Recently, KR Patent Application No. 10-2007-0084868 discloses that mice were immunized with a cholangiocarcinoma cell line to obtain a monoclonal antibody (A10-A3), this antibody was identified to specifically recognize L1CAM, L1CAM was identified to be expressed on the surface of cholangiocarcinoma cells, statistical analysis of correlation between L1CAM expression rate and survival rate in cholangiocarcinoma patients showed that the mortality of cholangiocarcinoma patients with a high L1CAM expression rate was much higher than that of the cholangiocarcinoma patients with a low L1CAM expression rate, which demonstrates that L1CAM serves as a poor prognostic factor for cholangiocarcinoma, and that *in vitro* and *in vivo* animal test results ascertained antibodies to L1CAM exhibit an inhibitory activity on growth, migration or invasion of cholangiocarcinoma cells, which indicates that L1CAM exhibits potent diagnostic and therapeutic effects on cholangiocarcinoma.

A great deal of recent research reported that L1CAM inhibits apoptosis of ovarian cancers by C2-ceramide, staurosporine, cisplatin and hypoxia (Stoeck A. et al Gynecol Oncol. 2007;104(2):461-469), and L1CAM increases in ovarian and pancreatic cancer cells treated with cisplatin.

However, none of these prior art references disclose that administration of an antibody which inhibits the action of L1CAM in combination with an antitumor agent causes synergistic cancer treatment efficacy.

The term "antitumor agent" used herein generically refers to a drug which acts on a variety of metabolic pathways of cancer cells and exhibits cytotoxicity or cytostatic effects on the cancer cells. Antitumor agents developed to date are classified into antimetabolites, plant alkaloids, topoisomerase inhibitors, alkylating agents, anticancer antibiotics, hormone preparations and other drugs, depending on mechanisms of action and chemical structure thereof. Antitumor agents have different intracellular targets, block DNA replication, transcription and translation of cells and suppress action of proteins essential for cell survival. Such an effect on intracellular targets causes cell death through the subsequent necrosis or apoptosis process. The mechanisms on which these antitumor agents act are not only specific for cancer cells, but also specific for normal cells, thus inevitably causing damage (i.e., toxicity) to normal tissues during administration of antitumor agents. However, there is a quantitative difference between tumor cell metabolism and normal cell metabolism. For this reason, antitumor agents are more toxic to tumor tissues. This selective toxicity of antitumor agents enables clinical chemotherapy. Antitumor agents having a higher therapeutic index are considered to be safe in that they eliminate tumor cells without imparting toxicity to normal tissues.

About 50 types of antitumor agents have been developed to date, the type of antitumor agents used is varied depending on the type and characteristics of tumors and an antitumor agent may be used alone or in combination with other chemotherapy for cancer treatment. The most commonly used antitumor agents are DNA alkylating agents (*e.g*., cyclophosphamide and ifosfamide), antimetabolites (*e.g*., methotrexate), folate inhibitors, pyrimidine inhibitors (*e.g.* 5-fluorouracil), microtubule inhibitors (*e.g.,* vincristine, vinblastine and paclitaxel), DNA intercalators (*e.g*., doxorubicin and cisplatin), hormone drugs (*e.g*., tamoxifen and flutamide) and the like.

Cisplatin was developed as an antitumor agent having cytotoxicity in 1968 and is known to conjugate with double strands of DNA and thus linked to adjacent DNA to prevent separation of DNA strands and thereby to inhibit cell division. Cisplatin is an antitumor agent which is most widely used, in particular, for the treatment of solid tumors such as ovarian cancer, testicular cancer and head and neck cancer. However, use of cisplatin is limited to cancers having resistance to cisplatin due to intrinsic or foreign factors (Andrews, P.A. and Howell, SB.(1990) Cancer cells.; Kelley, s.1. and Rozencweig, M (1989) Eur. J. Clin. Oncol. 25:1135-1140; Perez, R.P. et al. (1990) pharmacol. Ther. 48:19-27; and Timmer-Bosscha, H. et al. (1992) Br. J. Cancer 66:227-238). Accordingly, there is a demand for treatment via administration of cisplatin in combination with an inhibitor against resistance-inducing factors.

Gemcitabine (2'-deoxy-2',2'-difluorocytidine) known as a nucleoside analog is an antitumor agent which inhibits cell proliferation via synthesis of DNA and is used for the treatment of solid cancers such as bladder cancer, breast cancer, lung cancer, ovarian cancer, cholangiocarcinoma and pancreatic cancer. In spite of clinically superior efficacy, the concentration of gemcitabine to be used is extremely limited due to serious side effects such as anemia, leucopenia, neutropenia, an increase in liver enzymes, vomiting and fever. Accordingly, there is a demand for a combined drug in which the amount of gemcitabine is as low as possible and its therapeutic efficacy is improved.

5-fluorouracil known as a cytotoxic antimetabolite is an antitumor agent used for the treatment of solid cancers such as gastrointestinal cancer, breast cancer, and head and neck cancer and, in particular, is widely used in combination with other antitumor agents. Particularly, 5-fluorouracil is known to be considerably effective in treating solid cancers, when administered in combination with potassium salt of leucovorin or folinic acid (Malet-martino M et al. Clinical studies of three oral prodrugs of 5-fluorouracil (Capecitabine, UFT, S-1): A review. Oncologist 2002;4(4)228-323). However, 5-fluorouracil is only suitable for intravenous administration, since it exhibits cytotoxicity and low bioavailability, when orally administered. In addition, 5-fluorouracil is limited to the treatment of patients who exhibit strong activity on a dihydropyrimidine dehydrogenase (DPD) enzyme, since 85% or more is inactivated by the DPD enzyme. Accordingly, there is a demand for administration of 5-fluorouracil in combination with other antitumor agents, to improve the efficacy of 5-fluorouracil (Malet-martino M *et al*.).

Docetaxel (Taxol^{®}) and paclitaxel, a derivative thereof, are antitumor agents used for the treatment of solid cancers or highly metastatic malignant tumors, EP Patent 0253738 and International Publication No. WO 92/09589 disclose a method for preparing docetaxel. The amount of docetaxel used is 60 to 300 mg/m², which may be varied from patient to patient. Generally, docetaxel is intravenously administered at a level of 60 to 100 mg/m² for one hour or longer over three weeks (Textbook of Medical Oncology, Franco Cavelli et al., Martin Dunitz Ltd., p.4623 (1997)). A great deal of clinical research showed efficacies of docetaxel in treating breast cancer and the efficacies are known to be observed after one or two trials. The action modes of docetaxel are found to be an increase of microtubule assembly and suppression of depolymerization of tubulin.

### Disclosure

### Technical Problem

As a result of intensive studies to develop an active ingredient exhibiting potent effects as an antitumor agent, the present inventors discovered that antitumor agents exhibiting considerably superior effects can be provided by using a combination of an inhibitor of L1CAM activity or expression, each of which may be used singly, and a conventional antitumor agent. The present invention was completed based on this discovery.

### Technical Solution

In accordance with an aspect of the present invention, provided is an anticancer composition comprising an inhibitor of L1CAM activity or expression and an antitumor agent, which the anticancer composition exhibits synergistic therapeutic effects, compared to when each of them is administered alone, and is thus effective in treating cancers expressing L1CAM.

### Advantageous Effects

In the case where the anticancer composition comprising an inhibitor of L1CAM activity or expression and an antitumor agent according to the present invention is administered, the inhibitor of L1CAM activity or expression and the antitumor agent contained therein are simultaneously, separately or sequentially used in combination thereof. As a result, the composition exhibits considerably potent and significant effects on inhibiting growth and death of cancer cells, as compared to pharmaceutical effects via single administration of each of these substances, thus being considerably useful for the treatment of cancers, in particular, a variety of cancers, known as carcinomas expressing L1CAM, such as ovarian cancer, endometrial cancer, cervical cancer, breast cancer, colorectal cancer, melanoma, neuroblastoma, cholangiocarcinoma, lung cancer and pancreatic cancer.

### Description of Drawings

The above and other objects, features and other advantages of the present invention will be more clearly understood from the following detailed description taken in conjunction with the accompanying drawings, in which:
FIG. 1(A) shows analysis results of inhibitory activity on cell proliferation in cholangiocarcinoma cell lines (SCK) expressing L1CAM, to which antibody to L1CAM (A10-A3), cisplatin, or a combination of A10-A3 and cisplatin is administered, and FIG. 1(B) shows analysis results of cell death induction activity in cholangiocarcinoma cell lines (SCK) expressing L1CAM to which antibody to L1CAM (A10-A3), cisplatin or a combination of A10-A3 and cisplatin is administered;
FIG. 2 shows tumor growth in mice groups (n=10) to which mIgG, antibody to L1CAM (A10-A3), cisplatin, or a combination of A10-A3 and cisplatin is administered, in mouse xenograft models transduced with L1CAM-expressing cholangiocarcinoma cell lines (SCK);
FIG. 3(A) and 3(B) show analysis results of inhibitory activity on cell proliferation in cholangiocarcinoma cell lines (SCK) and ovarian cancer cell lines (SK-OV3), expressing L1CAM, to which after antibody to L1CAM (A10-A3), gemcitabine or a combination of A10-A3 and gemcitabine is administered;
FIG. 4(A) and 4(B) show analysis results of inhibitory activity on cell proliferation in cholangiocarcinoma cell lines (SCK) and ovarian cancer cell lines (SK-OV3), expressing L1CAM, to which antibody to L1CAM (A10-A3), 5-fluorouracil or a combination of A10-A3 and 5-fluorouracil is administered;
FIG. 5(A) shows comparison results of mRNA and protein levels of L1CAM expressed in cell lines wherein expression of L1CAM in L1CAM-expressing cholangiocarcinoma cell lines (SCK) is inhibited using siRNA specific for L1CAM, and control group cell lines;
FIG. 5(B) shows inhibitory activity on cell proliferation via co-administration of cisplatin in cell lines wherein expression of L1CAM in L1CAM-expressing cholangiocarcinoma cell lines (SCK) is inhibited using siRNA specific for L1CAM, and control group cell lines; and
FIG. 5(C) shows induction activity on cell death via co-administration of cisplatin in cell lines wherein expression of L1CAM in L1CAM-expressing cholangiocarcinoma cell lines (SCK) is inhibited using siRNA specific for L1CAM and control group cell lines.

### Best Mode

In accordance with one aspect to accomplish the objects above, the present invention is directed to an anticancer composition comprising, as active ingredients, the following ingredients (a) and (b) for simultaneous, separate or sequential use:
(a) an inhibitor of L1CAM activity or expression; and
(b) an antitumor agent.

In one embodiment, the present composition may comprise (a) an inhibitor of L1CAM activity. Preferably, the inhibitor of LICAM activity is an anti-L1CAM antibody which specifically recognizes L1CAM known as a cancer cell surface antigen or a secreted surface antigen. Such antibody includes all monoclonal antibodies, chimeric antibodies thereof and humanized antibodies. Novel antibodies, as well as antibodies well-known in the art, fall within the scope of the antibody of the present invention, which may be prepared by a method well-known in the art. For example, the antibody contained in the composition of the present invention may be easily prepared by a well-known method for preparing monoclonal antibody or a well-known method for chimerizing and humanizing monoclonal antibody. For example, the preparation of monoclonal antibody may be carried out by preparing hybridoma using B lymphocyte derived from immunized animals (Koeher and Milstein, 1976, Nature, 256:495), or by using phage display technology. The present invention is not limited to the preparation methods above.

Antibody library using phage display technology is a method in which antibody genes are directly obtained from B lymphocyte without preparing hybridoma to express antibodies on the phage surface. The use of phage display technology enables conventional difficulties associated with formation of monoclonal antibodies by B-cell immortalization to be avoided. A general phage display technology comprises: 1) incorporating an oligonucleotide having a random sequence into the gene site which corresponds to the N-terminal of phage coat protein pIII (or pIV); 2) expressing fused protein of a part of natural coat protein and polypeptide coded by the oligonucleotide with random sequence; 3) treating an acceptor bonded to the polypeptide coded by the oligonucleotide; 4) eluting peptide-phage particles bonded to the acceptor using a molecule having a low pH or bonding competitiveness; 5) amplifying the eluted phage in host cells by a panning process; 6) repeating the previous process to obtain a desired level; and 7) identifying a sequence of active peptide from DNA sequences of phage clones selected by panning.

Accordingly, the preparation of L1CAM-specific anti-L1CAM may be carried out by using a phage display technology in which antibody bonded to L1CAM is screened from a human antibody library by a panning process, or immunizing mice with L1CAM proteins to form human antibody library, or preparing hybridoma to prepare antibodies bonded to L1CAM. More preferably, the antibody is an anti-L1CAM antibody (A10-A3) disclosed in KR Patent No. 10-0756051 and/or KR Patent Laid-open No. 10-2008-0018149, and the A10-A3 antibody may be prepared in accordance with a method disclosed in the known documents, and is preferably secreted and prepared by hybridoma having an accession number of KCTC 10909BP.

As long as they have the binding specificity for L1CAM, the antibodies include complete forms having two full-length heavy chains and two full-length light chains and forms of functional antigenic fragments of antibody molecules. The term "functional antigenic fragments of antibody molecules" used herein refers to fragments retaining at least an antigen-binding function and examples thereof include Fab, F(ab'), F(ab')₂, Fv and the like.

In another embodiment, the antitumor composition may comprise (a) an inhibitor of L1CAM expression. When its expression level in L1CAM-expressing cancer cells is suppressed by an inhibitor of L1CAM expression, the action of L1CAM which mediates the growth and metastasis of cancer cells decreases, thus enabling cancer treatment. The inhibitor of L1CAM expression is preferably selected from the group consisting of siRNAs, shRNAs and antisense oligonucleotides, and is more preferably an siRNA containing a sequence of 5'-GCCAATGCCTACATCTACGTT-3' (Sequence ID No. 1).

The term "siRNA" used herein refers to a small nucleic acid molecule with a size of about 20 nucleotides, which mediates RNA interference or gene silencing. The term "shRNA" used herein refers to a short hairpin RNA in which sense and antisense sequences of a siRNA target sequence are arranged such that a loop structure consisting of 5 to 9 bases is interposed between. Recently, the RNA interference (RNAi) phenomenon has been studied for application to a method for controlling protein expression at the gene level. Typically, siRNA has been shown to inhibit protein expression by binding specifically to mRNA, having a sequence complementary to a target gene.

siRNA contained in the composition according to the present invention can be prepared by direct chemical synthesis (Sui G. et. al, (2002) Proc Natl Acad Sci USA 99:5515-5520) or synthesis using in vitro transcription (Brummelkamp T R et al., (2002) Science 296:550-553), but the present invention is not limited to these methods. Also, shRNAs are designed to overcome the drawbacks of siRNAs, including high biosynthesis costs and low transfection efficiency, leading to the short-term persistence of the RNA interference effect. shRNAs can be expressed from an RNA polymerase III-based promoter using an adenoviral, lentiviral or plasmid expression vector system incorporated into cells. The shRNA is well-known to be transformed into siRNA having a known structure by an siRNA processing enzyme (Dicer or RNase III) present in the cells and then induce silencing of a target gene

As used herein, the term "antisense" refers to an oligomer having a sequence of nucleotide bases and a subunit-to-subunit backbone that allows the antisense oligomer to hybridize with a target sequence in RNA by Watson-Crick base pairing to form an RNA:oligomer heteroduplex typically with mRNA within the target sequence. The oligomer may have an exact sequence complementary to the target sequence or near complementary thereto. These antisense oligomers may block or inhibit the translation of the mRNA, and/or modify the processing of mRNA to produce a splice variant of the mRNA. Thus, the antisense oligomer of the present invention is an antisense oligomer complementary to the mRNA of the L1CAM gene.

In addition, the composition of the present invention comprises (b) the antitumor agent administered in combination with (a).

The term "antitumor agent" herein used generically refers to a well-known drug used for the treatment cancer cells, which acts on a variety of metabolic pathways of cancer cells and exhibits cytotoxicity or cytostatic effects in cancer cells, and include all antitumor agents developed to date, including antimetabolites, plant alkaloids, topoisomerase inhibitors, alkylating agents, anticancer antibiotics, hormone preparations and other drugs.

The antitumor agents which are contained in the composition of the present invention and exhibit inhibitory effects on activity or expression of L1CAM are preferably cisplatin lines, such as cisplatin, carboplatin and heptaplatin, gemcitabine, 5-fluorouracil, and Taxol lines, such as docetaxel and derivatives thereof (paclitaxel). A particularly preferable antitumor agent is cisplatin. These antitumor agents may be prepared by a known method or be commercially available.

In the present invention, the inhibitor of L1CAM activity or expression and the antitumor agent may be simultaneously administered in combination thereof, or the substance and the antitumor agent may be separately simultaneously or sequentially administered, or separately administered at an interval. In the case of non-simultaneous administration, for example, the two active ingredients are alternately administered, or one is continuously administered and the other is then administered.
The composition of the present invention is not limited so long as it comprises, as active ingredients, an inhibitor of L1CAM activity or expression and an antitumor agent, and may be any type of drug. For example, the composition may be provided in the form of a combined or single preparation containing two active ingredients. The term "combined preparation" used herein refers to a preparation containing a mixture of two or more active ingredients in one formulation, and the term "single preparation" used herein refers to a preparation containing one active ingredient in one formulation. The active ingredients may be prepared and combined in the form of a formulation determined, depending on pharmaceutical and pharmacodynamic properties. In the present invention, a therapeutic agent in which the two active ingredients are provided in a single preparation refers to a drug wherein single preparations, each of which can be used singly, are combined. In the case where two active ingredients are provided in a separate dosage form, the preparation may be in the form of a kit comprising the two ingredients.

In addition, the anticancer composition of the present invention may be administered daily or intermittently and be administered once or several times every day. In the case where each of two active ingredients is provided in the form of a single preparation, the administration number thereof may be identical or different.

The anticancer composition of the present invention may be formulated alone or in combination with a suitable pharmaceutically acceptable carrier or excipient, as described below. Examples of formulations include oral formulations such as soft capsules, hard capsules, tablets, syrups, formulations for injection and formulations for external application.

The pharmaceutically acceptable carrier may include any of the known standard pharmaceutical carriers used for formulations such as sterilized solutions, tablets, coated tablets and capsules. Typically, the carrier includes excipients such as polyvinyl pyrrolidone, dextrin, starch, milk, sugars, specific types of clay, gelatin, stearate, talc, vegetable oils (*e.g*., edible oil, cotton seed oil, coconut oil, almond oil or peanut oil), oily ester such as neutral fatty acid glyceride, mineral oils, vaseline, animal fats and oils, cellulose derivatives (*e.g*., crystalline cellulose, hydroxypropyl cellulose, hydroxypropyl methylcellulose or methyl cellulose) and other well-known excipients. The carrier may further comprise an antioxidant, a wetting agent, a viscosity stabilizer, a flavor, a color additive and other ingredients. The composition containing the carrier may be formulated in accordance with a well-known method.

In addition, the pharmaceutical composition may be administered in a pharmaceutically effective amount for cancer treatment. The typical dosage and a ratio of (a) and (b) ingredients may be optimized using standard clinical technology.

In another aspect, the present invention is directed to a method for treating cancers using the anticancer composition.

Specifically, the treatment method of the present invention comprises administering a pharmaceutically effective amount of the anticancer composition to a patient. The anticancer composition may be administered parenterally, subcutaneously, intraperitoneally, intrapulmonarily or intranasally. For local therapy, the composition may be administered using a suitable method including intralesional administration, if desired. Parenteral injection includes intramuscular, intravenous, intraarterial, intraperitoneal and subcutaneous routes. Preferred administration modes include intravenous, subcutaneous, intradermal, intramuscular and drip injection.

As mentioned above, the dosage of the substances may vary depending on a variety of factors, and the administration route including oral and parenteral routes may also be determined depending on age of patients, administration period, weight, severity of diseases, consciousness of patients and the type of drugs co-administered. In addition, the substances may be separately administered simultaneously, sequentially or intermittently.

In another aspect, the present invention is directed to use of the anticancer composition for preparing a drug for treating cancers.

Specifically, examples of the cancer to be treated by the composition of the present invention include, but are not limited to, cholangiocarcinoma, endometrial cancer, cervical cancer, breast cancer, colorectal cancer, ovarian cancer, melanoma, neuroblastoma, lung cancer and pancreatic cancer.

The preparation of the drug for treating cancers may be carried out by the method described above, as well as technologies well-known in the art.

In another aspect, the present invention is directed to use of the anticancer composition for treating cancers.

Specifically, examples of the cancer to be treated by the composition of the present invention include, but are not limited to, cholangiocarcinoma, endometrial cancer, cervical cancer, breast cancer, colorectal cancer, ovarian cancer, melanoma, neuroblastoma, lung cancer and pancreatic cancer.

In one aspect, the inventors of the present invention administered a monoclonal antibody (A10-A3) inhibiting the action of L1CAM, and cisplatin, gemcitabine or 5-fluorouracil to L1CAM-expressing cancer cells, in order to confirm that proliferation of cancer cells was more efficiently inhibited, when an anti-L1CAM antibody and an antitumor agent are co-administered to the cancer cells, than when each of them is administered singly. As a result, it was confirmed that co-administration exhibited an inhibition of 80% or lower on proliferation of cancer cells, which indicates that co-administration exhibits potent synergistic effects as compared to single administration. In addition, in order to knock down expression of L1CAM in L1CAM-expressing cholangiocarcinoma cell lines, the cells were transduced with siRNA specific for L1CAM, cultured and then subjected to cell proliferation and cell death tests. As a result, it can be seen that, when expression of L1CAM in cholangiocarcinoma cell lines is inhibited, cisplatin inhibited cell proliferation and improved induction of cell death.

In the case where the anticancer composition comprising an inhibitor of L1CAM activity or expression and an antitumor agent according to the present invention is administered, the composition exhibits potent synergistic therapeutic effects, as compared to single administration of each of them, thus being considerably effective in treating a variety of cancers, in particular, carcinomas expressing L1CAM such as ovarian cancer, endometrial cancer, cervical cancer, breast cancer, colorectal cancer, melanoma, neuroblastoma, cholangiocarcinoma, lung cancer and pancreatic cancer.

Hereinafter, the present invention will be described in more detail with reference to the following Examples. These examples are only provided to illustrate the present invention and should not be construed as limiting the scope and spirit of the present invention.

### Mode for Invention

### Example 1. Culture of Cancer Cells

Carcinoma cell lines were cultured in an incubator at 37°C under 5% CO₂ using the following media containing 10% fetal bovine serum (Gibco Co. Ltd.). SCK (cholangiocarcinoma cell lines) and SK-0V3 (ovarian carcinoma cell lines) were cultured using a DMEM medium (Well GENE Co. Ltd.). SCK cell lines were obtained from Dr. Daegon Kim (Medical School, Chonbuk National University), and ovarian carcinoma cell lines were purchased from ATCC.

### Example 2. Synergic effects of co-administration of antibody to L1CAM and cisplatin on proliferation inhibition and death of cancer cells

Cell proliferation testing was performed in order to confirm that cell proliferation is more efficiently inhibited, when a monoclonal antibody (A10-A3) inhibiting the action of L1CAM and cisplatin are co-administered to L1CAM-expressing cancer cells, than when each of them is administered alone.

The A10-A3 antibody used as an anti-L1CAM antibody was prepared by a method disclosed in KR Patent Laid-open No. 10-2008-0018149.

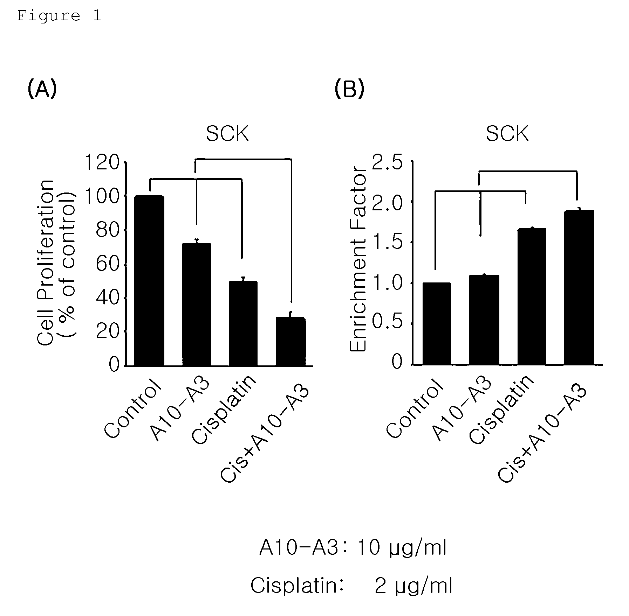

The respective cancer cells were incubated at a density of 2×10⁵ cells/well on 6-well plates containing 3 ml of a medium per well. The cells were treated with cisplatin or A10-A3 (10 µg/ml) alone or in combination thereof and were then cultured at 37°C in a CO₂ reactor for 72 hours. The cells were recovered, dead and live cells were counted using a 0.2% trypan blue solution and live cells of the total cells was expressed as a percentage. As a result, it could be seen that a group treated with cisplatin or A10-A3 (10 µg/ml) alone exhibited a 30-50% inhibition on proliferation of cancer cells, while a group treated with a combination of cisplatin and A10-A3 (10 µg/ml) exhibited a synergic inhibition effect of about 80% (at maximum) on proliferation of cancer cells (FIG. 1(A)).

In addition, cell death test was performed in order to confirm that co-administration of the antibody to L1CAM (A10-A3) and cisplatin causes more efficient induction of cell death, when co-administered to L1CAM-expressing cancer cells, than when each of them is administered alone. This test utilized the principle that DNA is fragmented when cells die. Respective cancer cell lines were incubated at a density of 2 × 10⁴ cells on 96 well plates containing a 100 µl medium, cisplatin or A10-A3 (10 µg/ml) was administered alone or in a combination thereof and cultured for 24 hours. After 24 hours, the medium was discarded, the cells were washed with a PBS buffer solution and were reacted with a cell lysis solution (RIPA buffer) at 37°C for 30 minutes. DNA-specific biotin was bonded to the cell solution, 20 µl of the resulting solution was transferred to 96-well plates containing streptavidin, 80 µl of immunoreagent was reacted therewith, and the absorbance of the resulting substance was measured at an OD of 405 nm. The result showed that cell death was remarkably increased, when A10-A3 and cisplatin were co-administered, as compared to when each was administered singly (FIG. 1(B)).

The results indicate that co-administration of antibody to L1CAM and cisplatin improves treatment efficacies on cancer cells.

### Example 3. Inhibitory effect of co-administration of A10-A3 and cisplatin on cancer growth in mouse animal model

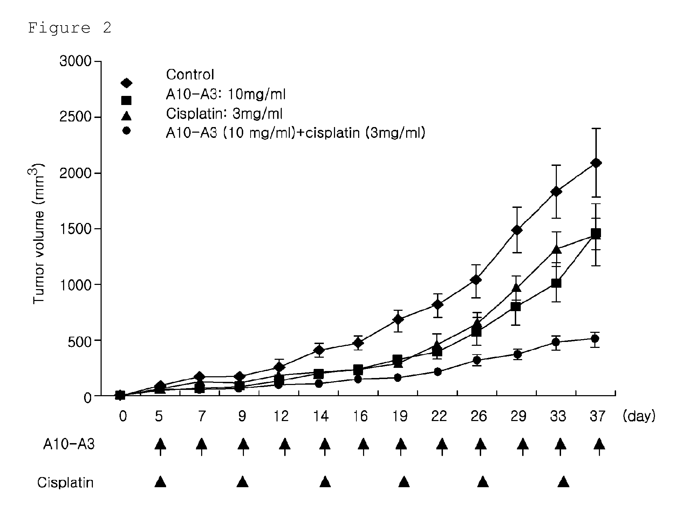

Nude mice Balb/c nu/nu were purchased via Central Lab. Animal Inc. from Japan SLC. The mice were 6 to 8 weeks old, weighed 18 to 22 g and acclimated for one week in a lab of the Korean Research Institute of Bioscience and Biotechnology. SCK cells (3×10⁷) were subcutaneously transplanted into 40 mice, and were randomly divided into four groups when grown to a tumor mass having a size of 100 to 200 mm³ after one week. A10-A3 was administered to the mice at a dose of 10 mg/kg three times per week. Cisplatin was administered to the mice at a dose of 3 mg/kg twice per week. As the control group, mIgG was administered at the same level, instead of A10-A3 and saline was administered at the same level, instead of cisplatin. The weight of nude mice and tumors were measured three times per week. As a result, the group to which A10-A3 or cisplatin was administered singly exhibited a proliferation inhibition of about 30%, while the group to which A10-A3 and cisplatin were co-administered exhibited remarkably superior inhibition of 70% or higher (FIG. 2).

### Example 4. Analysis of inhibitory effect of co-administration of antibody to L1CAM and gemcitabine on cancer cell proliferation

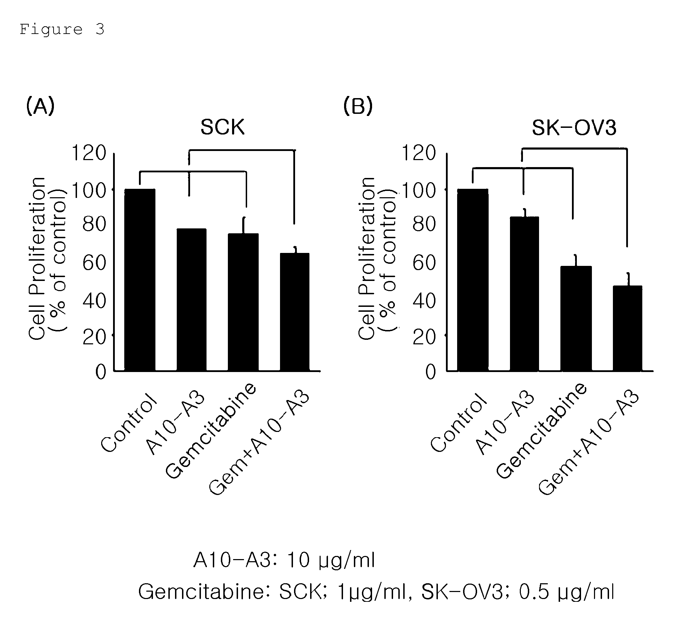

Whether co-administration of A10-A3 and gemcitabine more efficiently inhibits cell proliferation, as compared to single administration of A10-A3 or gemcitabine was analyzed in the same manner as in Example 2. As a result, it could be seen that the group wherein gemcitabine or A10-A3 (10 µg/ml) was administered singly exhibited a cancer cell proliferation inhibition of 20-25% (cholangiocarcinoma, SCK) or 20-40% (ovarian cancer, SK-OV3), while co-administration group exhibited an increased cancer cell proliferation inhibition of about 40-60% (FIG. 3(A)).

The results indicate that co-administration of antibody to L1CAM and gemcitabine improves treatment efficacies on cancer cells.

### Example 5. Analysis of inhibitory effect of co-administration of antibody to L1CAM and 5-fluorouracil on cancer cell proliferation

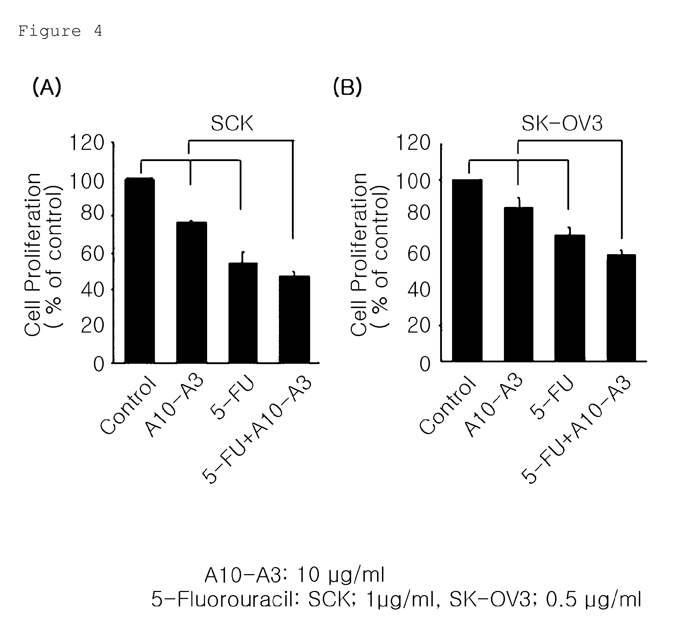

Whether co-administration of A10-A3 and 5-fluorouracil more efficiently inhibits cell proliferation, as compared to single administration of A10-A3 or 5-fluorouracil was analyzed in the same manner as in Example 2. As a result, it could be seen that the group to which 5-fluorouracil or A10-A3 (10 µg/ml) was administered singly exhibited a cancer cell proliferation inhibition of 20-40% (cholangiocarcinoma, SCK) or 20-30% (ovarian cancer, SK-OV3), while co-administration group exhibited an increased cancer cell proliferation inhibition of about 40-50% (FIG. 4)).

The results indicate that co-administration of antibody to L1CAM and 5-fluorouracil improves treatment efficacies on cancer cells.

### Example 6-1. Detection of mRNA expression of L1CAM by reverse transcriptase-polymerase chain reaction (RT-PCR)

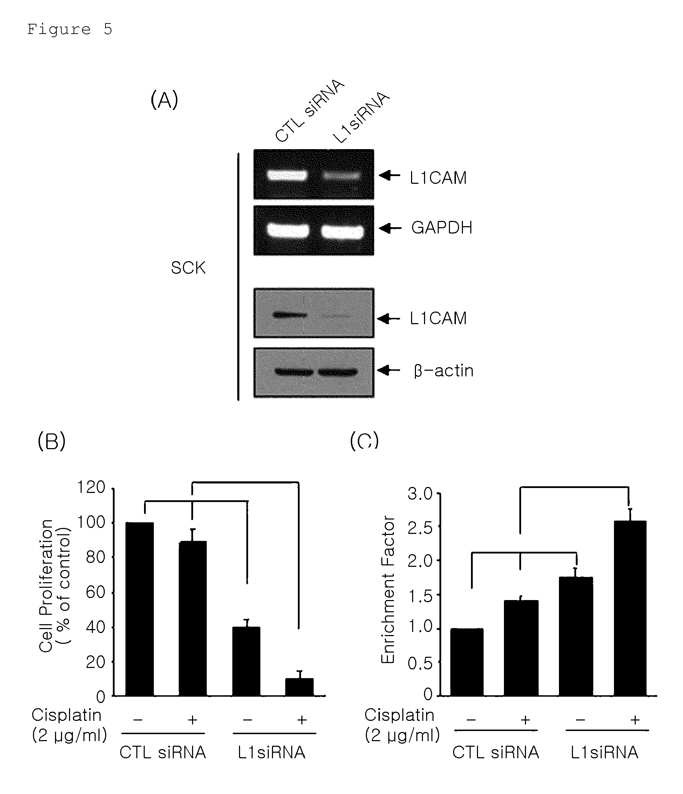

In order to knock down expression of L1CAM in cholangiocarcinoma cell lines (SCK), the cells were transduced with siRNA (5'-GCCAATGCCTACATCTACGTT-3', Sequence ID No. 1) specific for L1CAM and siRNA (5'-CAGTCGCGTTTGCGACTGG-3', Sequence ID No. 2) non-specific for L1CAM and then cultured for 72 hours. In order to isolate the total RNA of the cultured cholangiocarcinoma cells, the cells were treated with a 1 ml trizol reagent (Invitrogen, Inc.) at room temperature for 5 minutes. 0.2 ml of chloroform was added to the cells, and the mixture was violently shaken about 15 times and allowed to react at room temperature for 3 minutes. The resulting solution was centrifuged at 4°C and 14,000 rpm for 15 minutes, 400 µl of the supernatant was transferred to another tube and the same amount of isopropanol was added to the residue and the mixture was allowed to react at room temperature for 10 minutes. The reaction solution was centrifuged at 4°C at 14,000 rpm for 15 minutes, the supernatant was discarded, and the residue was washed with 75% ethanol. oligo(d)T, dNTP, RNase inhibitor and reverse transcriptase were added to 2 ug of the RNA thus extracted and the mixture was allowed to react at 42°C for one hour to synthesize cDNA. The cDNA was subjected to polymerase chain reaction (PCR) 30 times under the conditions of 30 sec/95°C, 30 sec/56°C and 30 sec/72°C using L1CAM primer, dNTP and Taq polymerase, and was then subjected to PCR at 72°C for 10 minutes. As a result, as compared to the control group treated with siRNA non-specific for L1CAM, the group treated with siRNA specific for L1CAM exhibited a decrease in the total amount of L1CAM expressed (upper view FIG. 5(A))

### Example 6-2. Detection of protein expression of L1CAM using Western blotting

In order to knock down expression of L1CAM in cholangiocarcinoma cell lines (SCK), the cells were transduced with siRNA (5'-GCCAATGCCTACATCTACGTT-3', Sequence ID No. 1) specific for L1CAM and siRNA (5'-CAGTCGCGTTTGCGACTGG-3', Sequence ID No. 2) non-specific for L1CAM and then cultured for 72 hours and expression of L1CAM was detected in protein level using Western blotting. In order to isolate the total protein of cell lines treated with siRNA, the cell lines were treated with 500 µl of cell lysis solution (RIPA buffer), and the proteins thus obtained were isolated in 10% SDS-PAGE, transferred to a nitrocellulose membrane and subjected to Western blotting. The nitrocellulose membrane was allowed to react in PBST (PBS + 0.1% Tween 20) buffer containing 5% skim milk for one hour and washed two or more times with the PBST buffer. Anti-L1CAM antibody (A10-A3) was added as a primary antibody to the reacted nitrocellulose membrane, followed by allowing to react for 2 hours. The resulting membrane was washed with the PBST buffer five times and reacted with anti-mouse IgG HRP (horseradish peroxidase) conjugate (1:5000 Sigma) for one hour. The reaction solution was washed five times with the PBST buffer and was colored with enhanced chemiluminescence reagent (ECL) (Amersham biosciences) as a detection reagent. As a result, as compared to the control group treated with siRNA non-specific for L1CAM, the group treated with siRNA specific for L1CAM exhibited a decrease in the total amount of protein expressed (lower view FIG. 5(A))

### Example 6-3. Analysis of effects of co-administration of siRNA inhibiting the expression of L1CAM and antitumor agent on cell proliferation inhibition and cell death induction

In order to knock down expression of L1CAM in L1CAM-expressing cholangiocarcinoma cell lines (SCK), the cells were transduced with siRNA (5'-GCCAATGCCTACATCTACGTT-3', Sequence ID No. 1) specific for L1CAM and siRNA (5'-CAGTCGCGTTTGCGACTGG-3', Sequence ID No. 2) non-specific for L1CAM and then cultured for 72 hours. Then, cell proliferation and cell death tests were performed in the same manner as in Example 2. As can be seen from FIG. 5(B) and 5(C), when expression of L1CAM in cholangiocarcinoma cell lines (SCK) was inhibited, cell proliferation inhibition and cell death induction effects of cisplatin were improved. These results indicate that, in addition to co-administration of antibody inhibiting the action of L1CAM and an antitumor agent, co-administration of siRNA inhibiting the expression of L1CAM and an antitumor agent also exhibited synergistic efficacy in cancer treatment.

### Industrial applicability

In the case where the anticancer composition comprising an inhibitor of L1CAM activity or expression and an antitumor agent according to the present invention is administered, the substance having inhibitory effects on activity or expression of L1CAM and the antitumor agent contained therein are simultaneously, separately or sequentially used in combination thereof. As a result, the composition exhibits considerably potent and significant effects on growth and death of cancer cells, as compared to pharmaceutical effects via single administration of each of these substances, thus being considerably useful for the treatment of cancers, in particular, a variety of cancers, known to be carcinomas expressing L1CAM, such as ovarian cancer, endometrial cancer, cervical cancer, breast cancer, colorectal cancer, melanoma, neuroblastoma, cholangiocarcinoma, lung cancer and pancreatic cancer.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An anticancer composition comprising, as active ingredients, the following (a) and (b) ingredients for simultaneous, separate or sequential use:
(a) an inhibitor of L1CAM activity or expression; and
(b) an antitumor agent,
wherein the inhibitor of L1CAM activity is selected from the group consisting of an anti-L1CAM antibody specific for L1CAM or an antigen-binding fragment thereof, and a variant of an anti-L1CAM antibody or an antigen-binding fragment thereof and the inhibitor of L1CAM expression is an oligonucleotide inhibiting L1CAM expression, and
the antitumor agent is selected from cisplatin, gemcitabine, 5-fluorouracil, docetaxel and paclitaxel.

2. The composition according to claim 1, wherein the oligonucleotide inhibiting L1CAM expression is selected from the group consisting of an antisense oligonucleotide, an siRNA and an shRNA to a gene encoding L1CAM.

3. The composition according to claim 1, wherein the anti-L1CAM antibody is an A10-A3 antibody secreted by hybridoma having an accession number of KCTC 10909BP.

4. The composition according to claim 2, wherein the siRNA has a sequence of SEQ ID NO: 1.

5. The composition according to any one of claims 1 to 4, wherein the antitumor agent is cisplatin.

6. The composition according to claim 1, wherein the composition inhibits growth of cancer cells or kills cancer cells.

7. The composition according to claim 1, wherein the cancer is selected from the group consisting of cholangiocarcinoma, endometrial cancer, cervical cancer, breast cancer, colorectal cancer, ovarian cancer, melanoma, neuroblastoma, lung cancer and pancreatic cancer.

8. The composition according to claim 1, wherein g each of the ingredients (a) and (b) is formulated in a separate dosage form.

9. A method for treating cancer comprising administering the anticancer composition according to claim 1.

10. The method according to claim 9, wherein the cancer is selected from the group consisting of cholangiocarcinoma, endometrial cancer, cervical cancer, breast cancer, colorectal cancer, ovarian cancer, melanoma, neuroblastoma, lung cancer and pancreatic cancer.

11. Use of the composition according to claim 1 for preparing a drug for treating cancer.

12. The use according to claim 11, wherein the cancer is selected from the group consisting of cholangiocarcinoma, endometrial cancer, cervical cancer, breast cancer, colorectal cancer, ovarian cancer, melanoma, neuroblastoma, lung cancer and pancreatic cancer.

13. Use of the composition according to claim 1 for treating cholangiocarcinoma, endometrial cancer, cervical cancer, breast cancer, colorectal cancer, ovarian cancer, melanoma, neuroblastoma, lung cancer or pancreatic cancer.
